# FASCICULE DE BREVET EUROPEEN

(11) **EP 0 899 330 B1**
(45) Date de publication et mention de la délivrance du brevet: **19.05.2004**
(21) Numéro de dépôt: 98402037.0
(22) Date de dépôt: 11.08.1998
(51) Int. Cl.: C12N 9/64, A61K 38/48, C07K 14/435, A61K 7/48

(54) **Polypeptide isolé de l'épiderme et son utilisation**
Aus dem Epidermis isolierte Polypeptide und Verwendung davon
Polypeptide isolated from the epidermis and its use

(30) Priorité: 29.08.1997 FR 9710818
(43) Date de publication de la demande: 03.03.1999
(73) Titulaire: L'OREAL, 75008 Paris (FR)
(72) Inventeur: Bernard, Dominique, 75015 Paris (FR); Kermici, Michel, 75012 Paris (FR); Bernard-Bourboulon, Marie-Alix, 75015 Paris (FR)
(74) Mandataire: Tezier Herman, Béatrice

(56) Documents cités:
- WO-A-95/07686
- WO-A-97/43425
- DE-A- 19 619 366
- GB-A- 2 306 961
- US-A- 5 710 014
- A. KAWADA ET AL.,: "Processing of cathepsins L, B and D in psoriatic epidermis" ARCHIVES OF DERMATOLOGICAL RESEARCH, vol. 289, no. 2, 1 janvier 1997, pages 87-93, XP002067217 BERLIN, DE
- S. GAL AND M. GOTTESMAN: "Isolation an sequence of a cDNA for human pro-(cathepsin-L)" THE BIOCHEMICAL JOURNAL, vol. 253, 1988, pages 303-306, XP002067218 LONDON, GB
- POPOVIC ET AL: 'Simultaneous isolation of human kidney cathepsins B,H,L and C and their characterisation' J. CHROMAT. BIOMED. APPL. vol. 681, no. 2, 1996, pages 251 - 262
- BIOSIS abstract PREV198783120860 Azaryan & Galoyan. 'Human and bovine brain cathepsin L and cathapsin H purification physicochemical properties and specificity' XP000926794 & Neurochem. Res. 1987, 12(2): 207-214.
- KAWADA ET AL: 'Rat epidermal cathepsin L-like proteinase: purification and some hydrolytic properties toward filaggrin and synthetic substrates' J. BIOCHEM. vol. 118, no. 2, 1995, pages 332 - 337
- DALTON ET AL: 'Induction of protective immunity in cattle against infection with Fasciola hepatica by vaccination with cathepsin L proteinases and with hemoglobin' INFEC. IMMUN. vol. 64, no. 12, Décembre 1996, pages 5066 - 5074
- DATABASE WPI Week 199432, Derwent Publications Ltd., London, GB; Class B04, AN 1994-260442 & JP 6 192 124 A (TORAY IND INC) 12 Juillet 1994

## Description

L'invention a pour objet un polypeptide isolé, un mélange de polypeptides issus de la protéolyse du polypeptide isolé, des compositions les contenant et un procédé de traitement cosmétique destiné à diminuer la cohésion intercornéocytaire, donc à favoriser la desquamation.

La peau constitue une barrière physique entre l'organisme et son environnement. Elle est constituée de deux tissus: l'épiderme et le derme.
Le derme fournit à l'épiderme un support solide. C'est également son élément nourricier. Il est principalement constitué de fibroblastes et d'une matrice extracellulaire composée elle-même principalement de collagène, d'élastine et d'une substance dite substance fondamentale, ces composants étant synthétisés par le fibroblaste. On y trouve aussi des leucocytes, des mastocytes ou encore des macrophages tissulaires. Il est également constitué de vaisseaux sanguins et de fibres nerveuses.

L'épiderme est un épithélium pluristratifié desquamant, de 100 µm d'épaisseur en moyenne et est conventionnellement divisé en une couche basale de kératinocytes qui constitue la couche germinative de l'épiderme, une couche dite épineuse constituée de plusieurs couches de cellules polyédriques disposées sur les cellules germinatives, une couche dite granuleuse constituée de cellules aplaties contenant des inclusions cytoplasmiques distinctes, les grains de kératohyaline, et enfin une couche supérieure appelée couche cornée (ou stratum corneum), constituée de kératinocytes au stade terminal de leur différenciation appelés cornéocytes. Ceux-ci sont des cellules momifiées, anucléées qui dérivent des kératinocytes.
Les cornéocytes sont principalement composés d'une matrice fibreuse contenant des cytokératines, entourée d'une structure très résistante de 15 nm d'épaisseur, appelée enveloppe cornée ou cornifiée. L'empilement de ces cornéocytes constitue la couche cornée qui est responsable de la fonction de barrière de l'épiderme.
Le stratum corneum possède une perméabilité sélective qui, en contrôlant la perte en eau, assure une hydratation physiologique de la peau. Il constitue de plus une barrière contre les agressions de l'environnement, qu'elles soient chimiques ou physiques.
Le stratum corneum se compose de deux parties :
- le stratum corneum compactum dont l'organisation cellulaire correspond à un empilement en colonne des cornéocytes au-dessus des cellules granuleuses dont ils sont issus. Chaque cornéocyte présente un recouvrement maximum avec les cornéocytes sus et sous-jacents.
- le stratum disjunctum constitué des dernières assises de la couche cornée, moins cohésives que les précédentes et lieu de la desquamation des cornéocytes.

Dans le stratum corneum l'espace intercornéocytaire est rempli par des feuillets lipidiques provenant des corps lamellaires.
La différenciation épidermique représente un processus de maturation continu et orienté qui, des kératinocytes basaux aboutit à la formation de cornéocytes, cellules mortes totalement kératinisées. Cette différenciation est la résultante de phénomènes parfaitement coordonnés qui vont conduire au maintien d'une épaisseur constante et assurer ainsi l'homéostasie de l'épiderme. Celle-ci passe par une régulation du nombre de cellules qui entrent dans le processus de différenciation et du nombre de cellules qui desquament.
Au cours du processus normal de desquamation, seuls les cornéocytes les plus superficiels se détachent de la surface de l'épiderme.
De la couche basale à la couche granuleuse, la cohésion est assurée par le réseau transcellulaire formé par les desmosomes et les filaments intermédiaires de cytokératines. Ce réseau est ancré sur la membrane basale par les hémidesmosomes.
Dans la couche cornée, la cohésion est assurée par des structures intercellulaires dérivant des desmosomes, appelées cornéosomes ou cornéodesmosomes, qui solidarisent les enveloppes cornées des cornéocytes. Dans l'épiderme (non palmo-plantaire), les cornéodesmosomes sont présents sur toute la surface cornéocytaire dans la partie inférieure de la couche cornée, mais seuls les cornéodesmosomes périphériques persistent dans la partie supérieure.
Des études récentes ont montré l'importance majeure des cornéodesmosomes dans la cohésion intercornéocytaire ainsi que dans le processus de desquamation. En particulier, une corrélation étroite existe entre la dissociation cellulaire et la protéolyse de certains composants cornéodesmosomaux comme la desmogléine I.
L'étude de la desquamation permet de mettre à jour l'existence d'une régulation biochimique fine jusque dans les couches dites «mortes» de l'épiderme. Ce sont des enzymes produites dans les couches vivantes plus profondes qui vont agir de façon séquentielle et complémentaire pour aboutir à la libération finale des cornéocytes à la surface cutanée.
Les principales enzymes soupçonnées de participer à la desquamation ont été décrites depuis peu. Elles appartiennent à deux familles d'enzymes : les glycosidases et les protéases. Les protéases ne peuvent agir seules et une action préalable de glycosidases démasquant des sites de protéolyse semble nécessaire.
Les protéases constituent le type d'enzymes qui est probablement le plus impliqué dans la desquamation. Elles se subdivisent en quatre familles:
- protéases à acide aspartique possédant un acide aspartique dans leur site actif,
- protéases à sérine avec une sérine dans leur site actif,
- protéases à cystéine comportant une cystéine dans leur site actif,
- métalloprotéases qui possèdent le plus fréquemment un atome de zinc dans leur site actif ou parfois un atome de calcium.

Parmi ces protéases, les protéases à cystéine d'origine lysosomiale (cathepsines B, H et L) sont sans doute les protéases les plus actives du corps humain. Ce sont elles qui participeraient au renouvellement quotidien des protéines d'un individu (de 200 à 300 g pour un individu de 70 kg).
A cet égard, le brevet n° WO 95/07686 décrit 2 protéases à cystéine de poids moléculaire apparent de 34 et 35 kilodaltons.

De nombreuses pathologies cutanées se caractérisent par la production d'une couche cornée épaissie et par une desquamation anormale, c'est-à-dire par une hyperkératose. Celle-ci peut survenir sur tout territoire anatomique cutané et dans des contextes cliniques très variés. Son substratum physiopathologique et sa cause sont variés.
A titre d'exemples on peut citer :
- la xérose (ou sécheresse cutanée),
- les ichthyoses,
- le psoriasis,
- certaines lésions tumorales bénignes ou malignes,
- les hyperkératoses réactionnelles.

D'autres pathologies se caractérisent par une transdifférenciation ou métaplasie, au niveau de muqueuses, malpighiennes ou non, mais normalement non cornifiées, qui deviennent cornifiées, c'est-à-dire se revêtent d'un épithélium anormal, producteur à sa surface d'une couche cornée. Bien que les muqueuses génitales et celles des voies aérodigestives supérieures soient le plus souvent concernées, ces métaplasies peuvent siéger dans divers territoires anatomiques.
A titre d'exemples on peut citer :
- la leukokératose du col utérin au cours du prolapsus,
- les leukokératoses buccales,
- les lésions tumorales bénignes kératosiques des muqueuses malpighiennes.

Sans vouloir se lier à une quelconque théorie de l'invention, il est possible de penser que ces pathologies peuvent être liées à un déficit, qualitatif ou quantitatif, en enzymes soupçonnées de participer à la desquamation dont en particulier des protéases.

La purification et la connaissance de polypeptides nouveaux impliqués dans la cohésion intercornéocytaire, en particulier de protéases, est une des voies qui pourrait permettre l'élaboration de nouveaux produits destinés à lutter contre les effets d'un excès ou d'un défaut en polypeptides, en particulier en protéases, principalement à la surface de la peau ou des muqueuses.

Un des objets de l'invention est donc de fournir sous forme isolée un polypeptide impliqué dans la cohésion intercornéocytaire.

Après de longs et laborieux travaux, la demanderesse a mis en évidence, isolé et purifié par des techniques biochimiques, à partir d'épiderme humain, un polypeptide impliqué dans la cohésion intercornéocytaire.

L'invention a donc pour objet un polypeptide isolé, appartenant à la famille des protéases à cystéine de type cathepsines L, ayant un poids moléculaire apparent de 28 kilodaltons et un point isoélectrique apparent compris entre 6 et 9.

On entend par poids moléculaire apparent, le poids moléculaire obtenu pour le polypeptide par comparaison de la mobilité électrophorétique de celui-ci avec celles de protéines standards de poids moléculaires connus sur gel de polyacrylamide/sodium dodecyl sulfate, ou encore par comparaison du volume d'élution du polypeptide avec celui de protéines standard de poids moléculaires connus en chromatographie d'exclusion (selon les techniques décrites dans "Protein Purification", J-C. Janson et L. Ryden, VCH Publisher Inc. N.Y., 1989).

On entend par point isoélectrique apparent le point isoélectrique obtenu pour le polypeptide par comparaison avec celui obtenu pour des protéines standards de point isoélectrique connus dans des expériences de chromatofocalisation (chromatofocusing) telles que décrites dans "Protein Purification", J-C. Janson et L. Ryden, VCH Publisher Inc. N.Y., 1989.

Le polypeptide de l'invention peut être d'origine naturelle ou synthétique.
Par synthétique, on entend ici tout polypeptide obtenu chimiquement ou par production dans un organisme après introduction dans cet organisme des éléments nécessaires à cette production.

Le polypeptide de l'invention peut être issu de toute origine possible à savoir soit animale, en particulier de mammifères et encore plus particulièrement humaine, soit végétale, soit de micro-organismes (virus, phages, bactéries entre autres) ou encore de champignons, sans préjuger du fait qu'il soit présent de manière naturelle ou non dans ledit organisme d'origine.

Préférentiellement, le polypeptide de l'invention est d'origine naturelle, isolé à partir de tissus de mammifères, particulièrement à partir de peau de mammifères.

Préférentiellement, le polypeptide de l'invention est isolé à partir de peau humaine et encore plus préférentiellement à partir d'épiderme humain.

Comme indiqué précédemment, la cohésion intercornéocytaire est apparemment due entre autres à l'existence dans la couche cornée, de polypeptides spécifiques des structures impliquées dans la jonction intercornéocytaire.

Ainsi, le polypeptide de l'invention est présent dans la couche cornée et il intervient dans la diminution de la cohésion intercornéocytaire en dégradant les structures impliquées dans la jonction intercornéocytaire, particulièrement des cornéodesmosomes.

Il est par ailleurs connu que les polypeptides et en particulier les enzymes, donc les protéases, peuvent se présenter sous une forme dite mature qui correspond à une séquence primaire en acides aminés compatible avec leur activité. Mais, l'on sait que souvent ces polypeptides matures sont issus, par phénomène de maturation, de polypeptides de taille supérieure, précurseurs, qui contiennent dans leur séquence primaire en acides aminés la séquence primaire du polypeptide mature. Il peut en être de même pour le polypeptide de l'invention.

Ainsi, l'invention a également pour objet tout polypeptide constitué en partie par le polypeptide de l'invention.

Il est connu également que les polypeptides peuvent subir des modifications post-traductionnelles comme la formation de liaisons disuifure, les clivages protéolytiques spécifiques, l'addition de glucides (glycosylation), la phosphorylation, en particulier au niveau des sérines et/ou des thréonines et/ou des tyrosines, et/ou l'association à des lipides.

L'invention concerne donc plus particulièrement le polypeptide de l'invention ayant subi ou non des modifications post-traductionnelles.

Le polypeptide de l'invention peut avoir subi une ou plusieurs modifications post-traductionnelles.

Préférentiellement, le polypeptide selon l'invention est glycosylé et/ou phosphorylé.

Le polypeptide de l'invention a un poids moléculaire apparent de 28 kilodaltons.

Il est bien connu que les enzymes en général, les protéases en particulier, présentent une activité maximale dans des milieux à pH défini.

Ainsi, le polypeptide de l'invention est caractérisé par le fait que son activité est maximale à un pH compris entre 2 et 9, préférentiellement entre 3,5 et 6,5.
Par exemple l'activité maximale du polypeptide sur la caséine est à un pH compris entre 4,6 et 5,6.

Il est également connu que la séquence primaire en acides aminés d'un polypeptide détermine des sites spécifiquement reconnus par des protéases qui une fois la reconnaissance de ces sites effective vont, avec ou sans fixation audit polypeptide, induire son clivage par protéolyse.

Ainsi, l'invention concerne également au moins un fragment de protéolyse du polypeptide de l'invention.

Il s'ensuit que par la suite dans le texte et sans indication contraire par polypeptide il faut entendre le polypeptide naturel ou synthétique de l'invention ou au moins l'un de ses fragments, qu'il soit obtenu par protéolyse ou de manière synthétique.

La cohésion intercornéocytaire est apparemment due à l'existence, dans la couche cornée, de polypeptides spécifiques des structures impliquées dans la jonction intercornéocytaire. On a vu que certaines pathologies hyperkératosiques pourraient être liées à un excès de cohésion intercornéocytaire.

La demanderesse a pu montrer que le polypeptide de l'invention intervient dans les phénomènes de destruction des structures impliquées dans la jonction intercornéocytaire donc dans la cohésion intercornéocytaire. Le polypeptide de l'invention peut donc être utilisé dans des compositions cosmétiques ou pharmaceutiques destinées à diminuer la cohésion intercornéocytaire et donc à favoriser la desquamation.

Un autre objet de l'invention est donc de fournir des compositions cosmétiques ou pharmaceutiques comprenant, dans un milieu physiologiquement acceptable, au moins un polypeptide tel que décrit précédemment.

De préférence, les compositions de l'invention sont appliquées sur la peau ou les muqueuses.

L'invention concerne également une composition pharmaceutique comprenant au moins un polypeptide de l'invention, destinée à traiter les troubles de la desquamation, comme les hyperkératoses, par exemple la xérose (ou sécheresse cutanée), les ichthyoses, le psoriasis, l'hyperkératose de certaines lésions tumorales bénignes ou malignes, les kératoses réactionnelles.

L'invention concerne également une composition pharmaceutique comprenant au moins un polypeptide de l'invention, destinée à traiter les pathologies se caractérisant par une transdifférenciation ou métaplasie, au niveau de muqueuses, malpighiennes ou non, mais normalement non cornifiées, qui deviennent cornifiées comme par exemple la leukokératose du col utérin au cours du prolapsus, les leukokératoses buccales, ou encore les lésions tumorales hyperkératosiques bénignes ou malignes des muqueuses malpighiennes.

La quantité de polypeptide contenue dans la composition de l'invention est bien entendu fonction de l'effet recherché et peut donc varier dans une large mesure.

Pour donner un ordre de grandeur la composition peut contenir le polypeptide de l'invention en une quantité représentant de 0,00001% à 50% du poids total de la composition et préférentiellement en une quantité représentant de 0,001% à 10% du poids total de la composition et encore plus préférentiellement en une quantité représentant de 0,1% à 1% du poids total de la composition.

Dans l'art antérieur, certains composés sont décrits comme des activateurs de protéase.
On connaît par exemple l'effet bénéfique du glycérol sur les xéroses, effet qui est expliqué par un effet activateur des systèmes enzymatiques, du à son action hydratante par laquelle il favoriserait l'action des protéases qui dégradent les cornéodesmosomes et par là même la desquamation (brevet N° WO 95/07687).

L'urée, et ses dérivés, est aussi connue depuis longtemps pour améliorer l'état de surface des peaux très sèches et même ichtyosiques (Swanbeck, Acta Dermatologica and Venereologica, 1968, 48, 123-127). Wiederanders et col. (Biomedical Biochemistry Acta, 1986, 45, (11-12), 1477-1483), ont montré qu'une cathepsine extraite de poisson est plus active en présence d'urée. Ces auteurs parviennent ainsi à doser spécifiquement les cathepsines L et D dont l'activité est respectivement multipliée par un facteur 2,5 et 6 en présence d'urée.

Des agents réducteurs sont également décrits comme activateurs des protéases. On citera par exemple les sulfures, les thiols comme le dithiothréitol ou le trithiohexitol, la cystéine, la N-acétylcystéine, les protéines ou les hydrolysats de protéines riches en cystéine, le mercaptoéthanol, le thioglycérol, les acides thioalcanoïques et les acides mercaptocarboxyliques et leurs analogues comme par exemple l'acide mercaptosuccinique, l'acide thiolactique, l'acide thioglycolique et leurs sels, le coenzyme A ou encore le glutathion réduit (GSH).
Ces agents réducteurs peuvent se trouver dans la composition sous leur forme active ou sous la forme de leur précurseur comme par exemple l'oxothiazolidine carboxylate qui est un précurseur des cystéines.

L'éthylène diamine tétraacétate (EDTA) est connu pour prévenir l'inactivation par les métaux lourds des protéases, particulièrement de type cathepsine. A ce titre l'EDTA est considéré comme un activateur de protéase.

On peut également assimiler les transglutaminases à un activateur de protéase. Ces enzymes appartiennent à la famille des transpeptidases. Elles sont calcium-dépendantes et catalysent la formation des ponts isopeptidiques ε(γ-glutamyl) Lysine : réaction du groupe carboxyle (sur le carbone γ) du résidu glutamine et du groupe aminé d'un résidu Lysine ou d'une polyamine. Les transglutaminases existent sous 2 formes principales dans l'épiderme : la transglutaminase E (ou épidermique), cytosolique, de PM 50-56 kD ayant un précurseur de 70 kD, et la transglutaminase K ou type I, membranaire, de PM 92 kD.
Les transglutaminases E et K sont toutes deux impliquées dans la formation de l'enveloppe cornée par pontage entre elles de nombreuses protéines, dont les principales sont l'involucrine, la loricrine, l'élafine, les cystatines, les pancornulines (ou SPR : Small Proline Rich ), des cytokératines, les desmoplakines I et II, des desmogléines et la cornéodesmosine.
Les cystatines sont des protéines possédant une activité inhibitrice des protéases à cystéine (Takahashi et col. FEBS letters, 1990, 2, 261-264).
Ainsi, si l'on augmente l'activité des transglutaminases, soit par la fourniture d'activateur de transglutaminase, soit par la fourniture directe de transglutaminase, on augmente alors la quantité de protéines constitutives de l'enveloppe cornée qui sont piégées dans la formation de cette dernière sous l'influence de la transglutaminase. On prive alors le stratum corneum de ses protéines endogènes, dont en particulier les cystatines. La disparition des cystatines dans l'épiderme et particulièrement dans le stratum cornéum, a alors pour effet de libérer les protéases à cystéine dont l'activité est alors augmentée ce qui a pour conséquence de diminuer la cohésion intercornéocytaire et donc de favoriser la desquamation.
Ainsi, l'invention à également pour objet une composition cosmétique ou pharmaceutique comprenant au moins un polypeptide selon l'invention et en outre au moins un activateur de protéase.

Parmi les activateurs de protéase on peut citer le glycérol, l'urée, l'EDTA, la transglutaminase, les agents réducteurs.

La quantité d'activateur de protéase contenue dans la composition de l'invention est bien entendu fonction de l'effet recherché et peut donc varier dans une large mesure.

Pour donner un ordre de grandeur la composition peut contenir l'activateur de protéase en une quantité représentant de 0,00001% à 15% du poids total de la composition et préférentiellement en une quantité représentant de 0,001% à 10% du poids total de la composition.

Dans la composition les activateurs de protéase peuvent être seuls ou en mélange.

Quelle que soit leur nature, les compositions de l'invention peuvent être ingérées, injectées ou appliquées sur la peau (sur toute zone cutanée du corps) ou les muqueuses (buccale, jugale, gingivale, génitale, conjonctivale, ...).

Selon le mode d'administration, les compositions selon l'invention peuvent se présenter sous toutes les formes galéniques normalement utilisées.

Pour une application topique sur la peau, la composition peut avoir la forme notamment de solution aqueuse ou huileuse ou de dispersion du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle du type crème ou gel aqueux ou anhydres, ou encore de microcapsules ou microparticules, ou de dispersions vésiculaires de type ionique et/ou non ionique ou de mousses ou encore sous forme de compositions pour aérosol comprenant également un agent propulseur sous pression. Ces compositions sont préparées selon les méthodes usuelles.

Pour l'injection, la composition peut se présenter sous forme de lotion aqueuse, huileuse ou sous forme de sérum. Pour les yeux, elle peut se présenter sous forme de gouttes et pour l'ingestion, elle peut se présenter sous forme de capsules, de granulés, de sirops ou de comprimés.

Les quantités des différents constituants des compositions selon l'invention sont celles classiquement utilisées dans les domaines considérés.

Ces compositions constituent notamment des crèmes de nettoyage, de protection, de traitement ou de soin pour le visage, pour les mains, pour les pieds, pour les grands plis anatomiques ou pour le corps (par exemple crèmes de jour, crèmes de nuit, crèmes démaquillantes, crèmes de fond de teint, crèmes anti-solaires), des fonds de teint fluides, des laits de démaquillage, des laits corporels de protection ou de soin, des laits anti-solaires, des laits après-soleil, des lotions, gels ou mousses pour le soin de la peau, comme des lotions de nettoyage, des lotions anti-solaires, des lotions après-soleil, des lotions de bronzage artificiel, des compositions pour le bain, des compositions désodorisantes comprenant un agent bactéricide, des gels ou lotions après-rasage, des crèmes épilatoires, des compositions contre les piqûres d'insectes, des compositions anti-douleur, des compositions pour traiter certaines maladies de la peau comme l'eczéma, la rosasée, le psoriasis, les lichens, les prurits sévères, l'ichthyose.

Les compositions selon l'invention peuvent également consister en des préparations solides constituant des savons ou des pains de nettoyage.

Les compositions peuvent aussi être conditionnées sous forme de composition pour aérosol comprenant également un agent propulseur sous pression.

La composition selon l'invention peut aussi être une composition pour les soins du cuir chevelu, et notamment un shampooing, une lotion de mise en plis, une lotion traitante, une crème ou un gel coiffant, une composition de teintures (notamment teintures d'oxydation) éventuellement sous forme de shampooings colorants, des lotions restructurantes pour les cheveux, une composition de permanente (notamment une composition pour le premier temps d'une permanente), une lotion ou un gel antichute, un shampooing antiparasitaire, les compositions antipelliculaires, etc.

La composition peut aussi être à usage bucco-dentaire, par exemple une pâte dentifrice. Dans ce cas, la composition peut contenir des adjuvants et additifs usuels pour les compositions à usage buccal et notamment des agents tensioactifs, des agents épaississants, des agents humectants, des agents de polissage tels que la silice, divers ingrédients actifs comme les fluorures, en particulier le fluorure de sodium, et éventuellement des agents édulcorants comme le saccharinate de sodium.

Lorsque la composition est une émulsion, la proportion de la phase grasse peut aller de 5 % à 80 % en poids, et de préférence de 5 % à 50 % en poids par rapport au poids total de la composition. Les huiles, les cires, les émulsionnants et les coémulsionnants utilisés dans la composition sous forme d'émulsion sont choisis parmi ceux classiquement utilisés dans le domaine cosmétique. L'émulsionnant et le coémulsionnant sont présents, dans la composition, en une proportion allant de 0,3 % à 30 % en poids, et de préférence de 0,5 à 20 % en poids par rapport au poids total de la composition. L'émulsion peut, en outre, contenir des vésicules lipidiques.

Lorsque la composition est une solution ou un gel huileux, la phase grasse peut représenter plus de 90 % du poids total de la composition.

De façon connue, la composition cosmétique peut contenir également des adjuvants habituels dans le domaine cosmétique, tels que les gélifiants hydrophiles ou lipophiles, les additifs hydrophiles ou lipophiles, les conservateurs, les antioxydants, les solvants, les parfums, les charges, les filtres, les absorbeurs d'odeur et les matières colorantes. Les quantités de ces différents adjuvants sont celles classiquement utilisées dans le domaine cosmétique, et par exemple de 0,01 % à 10 % du poids total de la composition. Ces adjuvants, selon leur nature, peuvent être introduits dans la phase grasse, dans la phase aqueuse et/ou dans les sphérules lipidiques.
Comme huiles ou cires utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles ou cires siliconées (cyclométhicone) et les huiles fluorées (perfluoropolyéthers), les cires d'abeille, de carnauba ou paraffine. On peut ajouter à ces huiles des alcools gras et des acides gras (acide stéarique). Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose^{R} 63 par la société Gattefosse.

Comme solvants utilisables dans l'invention, on peut citer les alcools inférieurs, notamment l'éthanol et l'isopropanol, le propylène glycol.
Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe, éthylcellulose, polyéthylène.

La composition peut contenir d'autres actifs hydrophiles comme les protéines ou les hydrolysats de protéines, les acides aminés, les polyols, l'urée, l'allantoïne, les sucres et les dérivés de sucre, les vitamines hydrosolubles, les extraits végétaux et les hydroxyacides.

Comme actifs lipophiles, on peut utiliser le rétinol (vitamine A) et ses dérivés, le tocophérol (vitamine E) et ses dérivés, les acides gras essentiels, les céramides, les huiles essentielles, l'acide salicylique et ses dérivés.

Selon l'invention la composition peut associer au moins un extrait d'au moins une Iridacée à d'autres agents actifs destinés notamment à la prévention et/ou au traitement des affections cutanées. Parmi ces agents actifs, on peut citer à titre d'exemple :
- les agents diminuant la différenciation et/ou la prolifération et/ou la pigmentation cutanée tels que l'acide rétinoïque et ses isomères, le rétinol et ses esters, la vitamine D et ses dérivés, les oestrogènes tels que l'oestradiol, l'acide kojique ou l'hydroquinone ;
- les antibactériens tels que le phosphate de clindamycine, l'érythromycine ou les antibiotiques de la classe des tétracyclines ;
- les antiparasitaires, en particulier le métronidazole, le crotamiton ou les pyréthrinoïdes ;
- les antifongiques, en particulier les composés appartenant à la classe des imidazoles tels que l'éconazole, le kétoconazole ou le miconazole ou leurs sels, les composés polyènes, tels que l'amphotéricine B, les composés de la famille des allylamines, tels que la terbinafine, ou encore l'octopirox ;
- les agents antiviraux tels que l'acyclovir ;
- les agents anti-inflammatoires stéroïdiens, tels que l'hydrocortisone, le valérate de bétaméthasone ou le propionate de clobétasol, ou les agents anti-inflammatoires non-stéroïdiens comme par exemple l'ibuprofène et ses sels, le diclofénac et ses sels, l'acide acétylsalicylique, l'acétaminophène ou l'acide glycyrrhizique ;
- les agents anesthésiques tels que le chlorhydrate de lidocaïne et ses dérivés ;
- les agents antiprurigineux comme la thénaldine, la triméprazine ou la cyproheptadine ;
- les agents kératolytiques tels que les acides α- et β-hydroxycarboxyliques ou β-cétocarboxyliques, leurs sels, amides ou esters et plus particulièrement les hydroxyacides tels que l'acide glycolique, l'acide lactique, l'acide salicylique, l'acide citrique et de manière générale les acides de fruits, et l'acide n-octanoyl-5-salicylique ;
- les agents hydratants comme le glycérol et ses dérivés ;
- les agents anti-radicaux libres, tels que l'α-tocophérol ou ses esters, les superoxyde dismutases, certains chélatants de métaux ou l'acide ascorbique et ses esters ;
- les antiséborrhéiques tels que la progestérone ;
- les antipelliculaires comme l'octopirox ou la pyrithione de zinc ;
- les antiacnéiques comme l'acide rétinoïque ou le peroxyde de benzoyle ;
- les extraits d'origine végétale ou bactérienne.

Ainsi, selon un mode particulier, la composition selon l'invention comprend également au moins un agent choisi parmi les agents antibactériens, antiparasitaires, antifongiques, antiviraux, anti-inflammatoires, antiprurigineux, anesthésiques, kératolytiques, anti-radicaux libres, anti-séborrhéiques, antipelliculaires, antiacnéiques et/ou les agents diminuant la différenciation et/ou la prolifération et/ou la pigmentation cutanée.

Un autre objet de l'invention est de proposer un procédé de traitement cosmétique pour lutter contre les excès de cohésion intercornéocytaire et donc pour augmenter la desquamation, procédé qui consiste à appliquer sur la peau une composition cosmétique comprenant au moins un polypeptide de l'invention.

L'invention a également pour objet l'utilisation du polypeptide de l'invention pour préparer ou purifier, éventuellement à partir d'épiderme, toute molécule, structurale ou fonctionnelle, susceptible de se lier spécifiquement audit polypeptide isolé ou auxdits fragments de protéolyse isolés ou audit peptide synthétique. Cette molécule peut notamment correspondre à d'autres protéines structurales spécifiques des cornéodesmosomes et diverses enzymes de la couche cornée, de type "protéases", "glycosidases" ou "phosphatases".

L'invention a également pour objet l'utilisation du polypeptide de l'invention pour préparer des antisérums et anticorps monoclonaux spécifiques, visant notamment à purifier cette protéine et ses fragments. Par extension, l'invention a également pour objet toute utilisation dudit polypeptide pour produire des anticorps ou fragments d'anticorps recombinants, quel que soit le système biologique utilisé pour produire ces derniers.

La figure 1 représente les profils d'activité des protéases contenues dans les échantillons de protéines du stratum corneum en présence ou en absence de cystéine.
La figure 2 représente les profils d'activité des protéases du pic G4 en fonction du pH.
La figure 3 représente les profils d'activité des protéases du pic G4 en présence de l'inhibiteur E 64 (Proteolytic enzymes : a practical approach, R.J. Beyton et J.S. Bond, IRL press, Oxford, 1989).
La figure 4 représente les profils d'activité des protéases du pic G4 en présence de leupeptine.
La figure 5 représente les profils d'activité des protéases du pic G4 en présence de chymostatine.
La figure 6 représente les profils d'activité des protéases du pic G4 en présence de l'inhibiteur CA 074 (Inubushi et col., J. of Biochemistry, 116, 282-284, 1994).
La figure 7 représente les profils d'activité des protéases du pic G4 en présence de pepstatine.
La figure 8 représente les profils d'activité des protéases du pic G4 en présence d'un substrat peptidique préférentiel des cathepsines L.
La figure 9 représente les profils obtenus lors des expériences chromatofocalisation pour la détermination du point isoélectrique apparent.

### EXEMPLE :

### Isolement et caractérisation du polypeptide :

Les protéines du stratum corneum sont prélevées par le procédé dit de «grattage». Cette technique ne nécessite pas de phase d'extraction par solvant organique et donc est susceptible de moins détruire les activités enzymatiques. Elle permet en outre d'obtenir de bonne quantité de matériel.
Le grattage est effectué sur la face antérieure de la jambe. La zone de prélèvement est lavée avec 200 ml d'un tampon constitué de 50 mM de tampon phosphate de sodium, pH 7, 5 mM d'EDTA, 150 mM de NaCl et 0.1 % de triton X100, distribué au moyen d'une pompe à débit constant de 100 ml/min.
La zone est alors grattée superficiellement avec le bord d'une lame de microscope.
Le liquide contenant les cellules est recueilli dans un récipient situé sous la jambe. Le tampon ainsi récolté est recyclé pour de nombreux passages (15). Les échantillons sont ensuite regroupés ou non selon le type d'expérimentation.
La solution ainsi obtenue est d'abord filtrée sur papier Whatman n°4, puis sur filtre Millipore 0.45 µm et enfin sur filtre Millipore 0.22 µm. Cette solution clarifiée est concentrée jusqu'à 12 ml par ultrafiltration tangentielle avec un seuil de coupure de 10 kD à 4°C avec une contre-pression de 1 bar et un débit de sortie de filtrat de 2 ml/min. au moyen de l'appareil K.BL™ et des membranes Sartocon™ (SARTORIUS).

Pour un individu peau saine, la concentration des protéines extraites par cette méthode est de l'ordre de 0.15 mg/ml pour 12 ml final avec 15 passages pour la technique.

### Séparation des protéines :

Une colonne Superdex G200 HR10/30™ (Pharmacia Biotech) est utilisée pour la séparation des protéines avec un domaine de résolution allant de 600 kD à 10 kD. Cette technique basée sur la séparation en fonction du poids moléculaire permet d'avoir une première estimation des poids moléculaires des protéines.
Le profil de chromatographie obtenu montre que les pics protéiques se situent majoritairement vers les bas poids moléculaires (20 à 40 kD).
Une colonne plus résolutive est alors utilisée. L'échantillon (250 µl) est injecté sur une colonne d'exclusion Superdex G75 HR10/30™ (Pharmacia Biotech) pour laquelle la séparation optimale se situe pour des poids moléculaires allant de 70 kD à 3 kD.
La sortie de colonne est collectée sur plaque 96 puits, à 4°C, à raison de 150 µl /puits, après un temps d'attente de 15 minutes après injection (Ce dernier correspond à un peu moins que le volume mort de la colonne et laisse une marge de sécurité pour le cas où un composé ne serait absolument pas retenu) et un volume de mort de 0,24 ml. Le débit de la pompe est de 0.5 ml/mn, la longueur d'onde de détection de 280 nm (Pompe série 10 : Perkin Elmer, Détecteur SP8450: Spectra Physics, Intégrateur LCI-100 : Perkin Elmer, Collecteur de fraction modèle 201 : Gilson).
Les profils chromatographiques obtenus sur différentes injections étant reproductibles, les fractions obtenues sont mélangées «puits à puits» afin d'avoir un volume de travail d'environ 800µl par fraction.
Les différentes fractions sont maintenues au froid, réfrigérateur ou glace pilée, afin de conserver les activités enzymatiques.
Les poids moléculaires correspondants au différentes fractions sont déterminés par une électrophorèse sur gel de polyacrylamide avec un gradient d'acrylamide 8-18 %. Les échantillons sont dilués au ¼ dans un tampon Laemmli modifié (0.0625 M Tris, pH 6.8, 2% SDS et sans DTT) et les dépôts sont de 20 µl. Les bandes protéiques sont révélées par une coloration au nitrate d'argent selon le protocole Pharmacia Biotech. (Kit: Silver staining plusone™).

### Dosage des activités protéasiques :

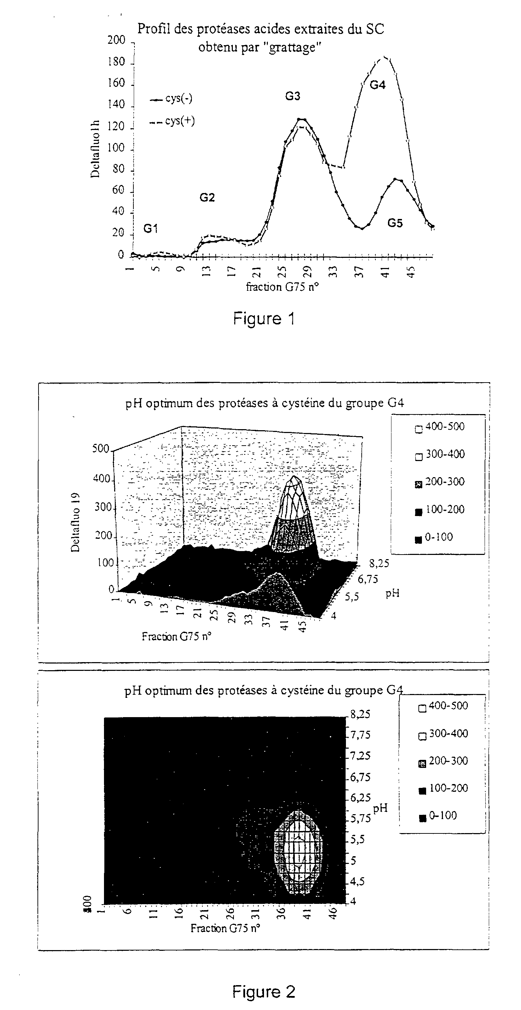

Un dosage des activités protéasiques contenues dans les fractions obtenues par passage sur la colonne d'exclusion Superdex G75 HR10/30™ est effectué par fluorimétrie à l'aide du kit Enzcheck™ (Molecular Probes).
Ce kit est un procédé rapide et simple de mesure des activités protéasiques, sans phase de précipitation ni de séparation et est donc adapté pour un criblage rapide des activités protéasiques des différentes fractions. Ce protocole utilise comme substrat le BODIPYfl-caséine™ qui devient fluorescent après digestion enzymatique. La fluorescence ainsi libérée est directement proportionnelle à l'activité protéasique contenue dans l'échantillon. La fluorescence est mesurée sur le spectrofluorimètre LS50B, Perkin Elmer, avec une longueur d'onde d'excitation de 485 nm (fente de 2.5 nm), une longueur d'onde d'émission de 535 nm (fente de 8 nm ) et un temps d'intégration de 1 seconde par puits.
Les dosages sont effectués en présence ou en absence de cystéine à 5 mM en concentration finale afin de mettre en évidence d'éventuelles activités de protéases à cystéine.
Les résultats sont montrés dans la figure 1.

Le profil protéasique révèle l'existence d'un pic (G4) présent lorsque le tampon contient de la cystéine et absent lorsque le tampon ne contient pas de cystéine.
Ce pic révèle donc dans les fractions 34 à 47 l'existence d'une protéase cystéine dépendante. Cette protéase a un poids moléculaire de l'ordre de 28 kD.

### pH optimal d'activité des protéases :

Pour caractériser le pH optimal d'activité des protéases du pic G4, deux tampons, tels que décrits dans "Data for Biochemical Research", (Dawson et col., 3^{ième} édition, Oxford science publications, 1990), ont été préparés pour couvrir une gamme de pH allant de 4,0 à 8.25 :
tampon acétate 0,1 M, pH 4,0 à 5,75 ;
tampon phosphate 0.1 M, pH 5,75 à 8,25.
Tous les tampons contiennent 5 mM d'EDTA et 0,1 % de triton X100. Les activités sont mesurées toutes les 0.25 unités de pH.
La technique précédente de mesure de l'activité protéasique est utilisé avec chacun des 2 tampons, sur chaque fraction.
Les résultats sont montrés dans la figure 2.
Le pH optimal des protéases extraites du stratum corneum, agissant sur le substrat BODIPYfl-caséine™ se situe dans les pH acides, de 4,0 à 6,25.

La plus forte activité a été obtenue pour entre les pH 5,0 et 5,5 avec le tampon acétate. Le tampon acétate à pH 5,0 est donc le tampon utilisé pour la suite des expérimentations.

### Caractérisation des pics de protéases par l'utilisation d'inhibiteurs :

Afin de mieux caractériser la protéase cystéine dépendante isolée précédemment, des tests d'inhibition par des inhibiteurs connus des protéases sont réalisés.
Les mesures sont effectuées de manière identique aux mesures précédentes en présence de cystéine à 5 mM en concentration finale et en présence ou en absence (témoin) de l'inhibiteur considéré.

### Inhibiteur E 64 :

E 64 est un inhibiteur des protéases à cystéine et en particulier des cathepsines B, H, L (Proteolitic enzymes : a practical approach, R.J. Beyton et J.S. Bond, IRL press, Oxford, 1989).

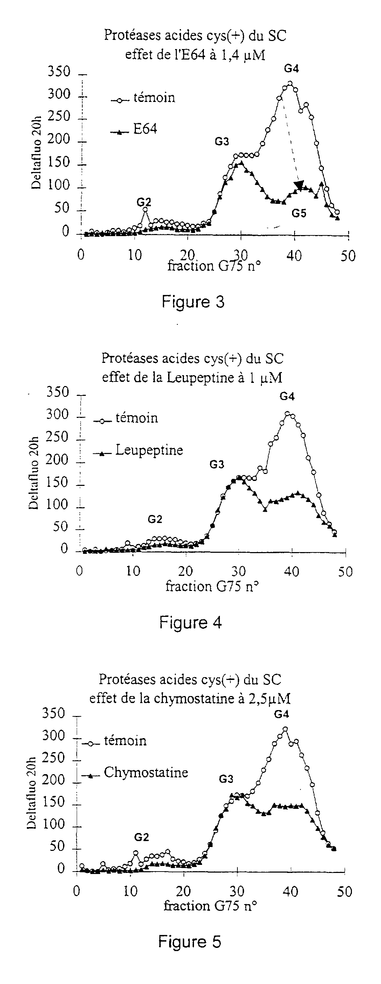

Le test est réalisé à une concentration de 1,4 µM en inhibiteur E64.
Les résultats sont montrés dans la figure 3.
L'E64 à 1,4 µM inhibe à environ 71% l'activité de la protéase cystéine dépendante du pic G4.
Au regard de la spécificité de cet inhibiteur, G4 est une cathepsine B, H ou L.

### Leupeptine :

La leupeptine, à la dose utilisée, est spécifique des cathepsines B ou L et sans effet sur les cathepsines H (Schwartz et col. 1980).
Le test est réalisé à une concentration de 1 µM en leupeptine.
Les résultats sont montrés dans la figure 4.
La Leupeptine à 1 µM, en présence de cystéine réduit d'environ 41% l'activité protéasique de G4.
G4 n'est pas une cathepsine H.

### Chymostatine :

La chymostatine est spécifique des cathepsines L et inactive à la concentration utilisée sur les cathepsines B (Inubushi et col., J. of Biochemistry, 116, 282-284, 1994).
Le test est réalisé à une concentration de 2,5 µM en chymostatine.
Les résultats sont montrés dans la figure 5.
Utilisée à 2.5 µM, la chymostatine inhibe à plus de 50 % la protéase cystéine dépendante de pic G4.
G4 est une protéase du type cathepsine L.

### Inhibiteur CA 074 :

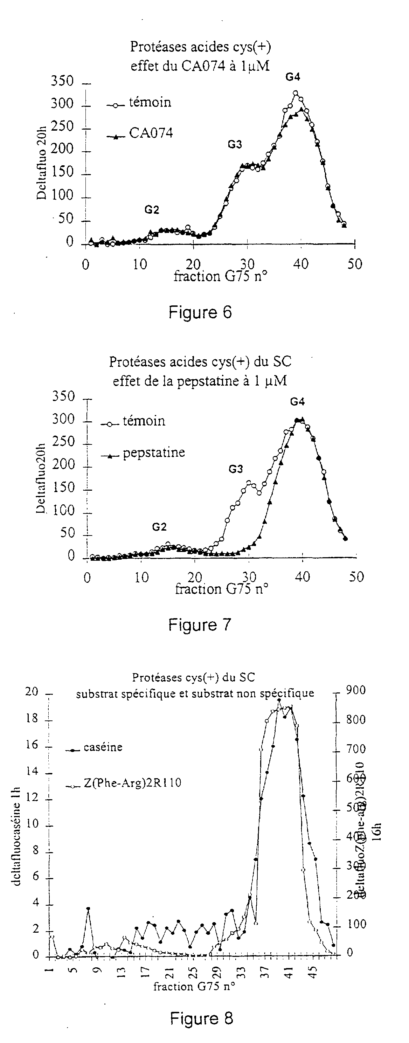

L'inhibiteur CA 074 inhibe spécifiquement les cathepsines B et est sans action sur les cathepsines L (Inubushi et col. 1994).
Le test est réalisé à une concentration de 1 µM en chymostatine.
Les résultats sont montrés dans la figure 6.
L'inhibiteur CA 074 n'affecte pratiquement pas le profil d'activité de la protéase cystéine dépendante de pic G4.
G4 n'est pas une protéase du type cathepsine B.

### Pepstatine :

La pepstatine est un inhibiteur spécifique des protéases à acide aspartique (Proteolytic enzymes : a practical approach, R.J. Beyton et J.S. Bond, IRL press, Oxford, 1989).

Le test est réalisé à une concentration de 1 µM en pepstatine.
Les résultats sont montrés dans la figure 7.
La pepstatine est sans action sur G4.
G4 n'est pas une protéase à acide aspartique.

### Utilisation d'un substrat spécifique des cathepsines L [Z(phe-arg 2R110) (Assfalg-Machleidt et col. 1992)] pour confirmer la caractérisation de G4 :

Les résultats d'identification de G4, obtenus par les différents tests inhibiteurs, ont été vérifiés par l'utilisation d'un substrat peptidique préférentiel des cathepsines L marqué à la rhodamine 110. Ce substrat est 850 fois plus sensible à l'action des cathepsines L que des cathepsines B.
Les résultats du bilan d'activité protéases en présence de cystéine, témoin sans cystéine soustrait, sont présentés dans la figure 8.
Les résultats montrent que l'hydrolyse intense du substrat peptidique spécifique se superpose parfaitement à celle de la caséine, confirmant la nature cathepsine L de G4.

### Evaluation du point isoélectrique apparent de G4 :

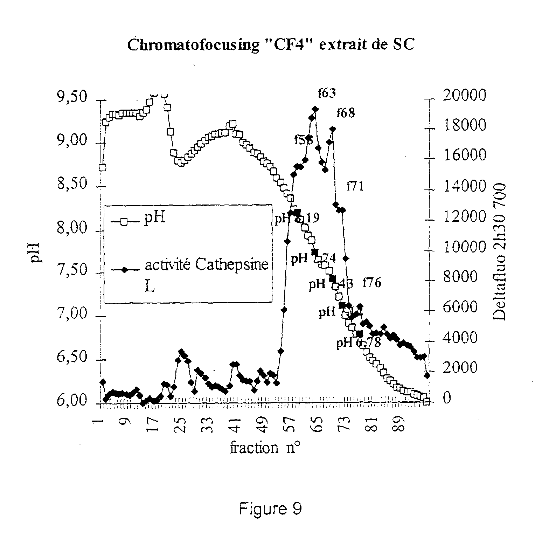

Cette évaluation est réalisée par la technique de chromatofocalisation (chromatofocusing) telles que décrites dans "Protein Purification", J-C. Janson et L. Ryden, VCH Publisher Inc. N.Y., 1989, dans les conditions suivantes :
Colonne : Mono PTM HR 5/20 Pharmacia.
Echantillon : extrait de SC humain équilibré dans un tampon 0.075M tris/acétate, pH 9.3).
Tampon d'élution : 10 ml polybuffer 96/acétate, pH 6.0, ("Protein Purification", J-C. Janson et L. Ryden, VCH Publisher Inc. N.Y., 1989).
Débit : 1ml/min.
Fractions : 0,5 ml à partir de l'injection sur 48 ml totaux.
Détection : Activités cathepsine L sur Z(phe-Arg)2R110 10 mM dans tampon acétate 0,1M pH5,0, 0,1% Triton X100, 5mM EDTA, 5mM cystéine. Incubation de 10µl de fraction + 200 µl de substrat à 37°C pendant 2h30. Lecture sur BioluminTM de Molecular Dynamics dont le photomultiplicateur est réglé à 700V, excitation : 485/10 nm, émission : 520/10 nm.
Les résultats sont présentés dans la figure 9.
Ces résultats montrent un point isoélectrique apparent compris entre 6 et 9.

### Mise en évidence du rôle probable de G4 dans le processus de desquamation :

La cornéodesmosine est une protéine essentielle du cornéodesmosome qui est dégradée lors de la desquamation (Serre G. et col. J.I.D., 1991, 97(6), 1061-1072).
Le test est pratiqué sur de la cornéodesmosine extraite de stratum corneum selon la technique développée par Munerot C. (Rapport de DEA., 1996, Université de Marne la Vallée).
Un immunotransfert pratiqué sur de la cornéodesmosine après incubation sur stratum corneum entier en présence ou en absence de la protéase du pic G4 montre une dégradation prononcée de celle-ci.
La protéase du pic G4 présente dans le stratum corneum dégrade la cornéodesmosine.

L'ensemble des résultats des essais pratiqués permet de conclure que le polypeptide extrait du stratum corneum est une protéase cystéine dépendante de type cathepsine L ayant un poids moléculaire apparent de 28 kD et un point isoélectrique compris entre 6 et 9.

## Revendications

1. Composition cosmétique ou pharmaceutique comprenant, dans un milieu physiologiquement acceptable, au moins un polypeptide ou au moins un fragment d'un polypeptide naturel ou synthétique isolé, appartenant à la famille des protéases à cystéine de type cathepsines L, ayant un poids moléculaire apparent de 28 kilodaltons et un point isoélectrique compris entre 6 et 9, ledit polypeptide pouvant être isolé à partir de la couche cornée de l'épiderme humain et ses fragments, **caractérisée par le fait que** ledit polypeptide intervienent dans la diminution de la cohésion intercornéocytaire en dégradant la cornéodesmosine.

2. Composition selon la revendication précédente, **caractérisé par le fait que** l'activité du polypeptide est maximale à un pH compris entre 2 et 9, préférentiellement entre 3,5 et 6,5.

3. Composition **caractérisée en ce qu'**elle comprend au moins un polypeptide constitué en partie par le polypeptide tel que décrit dans les revendications 1 à 2.

4. Composition selon l'une des revendications précédentes **caractérisée en ce qu'**elle contient au moins un fragment du polypeptide tel que décrit dans les revendications 1 à 2 obtenu par protéolyse ou de manière synthétique.

5. Composition selon l'une des revendications précédentes, **caractérisée par le fait que** le polypeptide ou le fragment est en une quantité comprise entre 0,00001% et 50%, de préférence comprise entre 0,001% et 10% en poids par rapport au poids total de la composition.

6. Composition selon l'une quelconque des revendications précédentes, **caractérisée par le fait qu'**elle comprend en outre au moins un activateur de protéase.

7. Composition selon la revendication 6 **caractérisée par le fait que** l'activateur de protéase est choisi parmi le glycérol, l'urée et ses dérivés, la transglutaminase, l'EDTA, les agents réducteurs.

8. Composition selon la revendication 7 **caractérisée en ce qu'**elle comprend au moins un activateur de protéases qui est un agent réducteur sous forme active ou sous forme de leur précurseur, choisi parmi les sulfures, les thiols comme le dithiothréitol ou le trithiohexitol, la cystéine, la N-acétylcystéine, les protéines ou les hydrolysats de protéines riches en cystéine, le mercaptoéthanol, le thioglycérol, les acides thioalcanoïques et les acides mercaptocarboxyliques et leurs analogues, l'acide mercaptosuccinique, l'acide thiolactique, l'acide thioglycolique et leurs sels, le coenzyme A, le glutathion réduit (GSH) ou l'oxothiazolidine carboxylate.

9. Composition selon l'une quelconque des revendications 6 à 8, **caractérisée par le fait que** l'activateur de protéase est en une quantité comprise entre 0,00001% et 15% et de préférence comprise entre 0,001% et 10% en poids par rapport au poids total de la composition.

10. Utilisation d'un polypeptide ou d'au moins un fragment d'un polypeptide naturel ou synthétique isolé, appartenant à la famille des protéases à cystéine de type cathepsines L, ayant un poids moléculaire apparent de 28 kilodaltons pour la préparation d'une composition cosmétique selon l'une des revendications 1 à 10, **caractérisée en ce que** la composition est destinée à être appliquée sur la peau pour lutter contre les excès de la cohésion intercornéocytaire ou pour favoriser la desquamation.

11. Composition pharmaceutique selon l'une quelconque des revendications 1 à 9, destinée à traiter les troubles de la desquamation.

12. Composition pharmaceutique selon la revendication 11 destinée à traiter l'hyperkératose.

13. Composition pharmaceutique selon l'une quelconque des revendications 11 ou 12, destinée à traiter la xérose, les ichthyoses, le psoriasis, les lésions tumorales bénignes ou malignes ou les kératoses réactionnelles.

14. Composition pharmaceutique selon l'une quelconque des revendications 11 ou 12, destinée à traiter la leukokératose du col utérin au cours du prolapsus, les leukokératoses buccales, ou encore les lésions tumorales bénignes kératosiques des muqueuses malpighiennes.

15. Polypeptide naturel ou synthétique isolé, appartenant à la famille des protéases à cystéine de type cathepsines L, ayant un poids moléculaire apparent de 28 kilodaltons, tel que décrit dans une composition selon l'une des revendications précédentes, **caractérisé en ce qu'**il est susceptible d'être isolé du stratum corneum humain et qu'il a un point isoélectrique compris entre 6 et 9.

16. Utilisation du polypeptide isolé ou de ses fragments tels que décrits dans les revendications 1 à 15, pour préparer ou purifier toute molécule susceptible de se lier spécifiquement audit polypeptide isolé ou auxdits fragments de protéolyse isolés ou audit peptide synthétique.

17. Utilisation selon la revendication précédente pour préparer ou purifier des protéines structurales spécifiques de cornéodesmosomes.

18. Utilisation du polypeptide isolé tel que décrit dans les revendications 1 à 4 ou 15, pour préparer ou purifier des antisérums ou des anticorps monoclonaux spécifiques.

## Patentansprüche

1. Kosmetische oder pharmazeutische Zusammensetzung, die in einem physiologisch akzeptablen Medium mindestens ein isoliertes natürliches oder synthetisches Polypeptid oder mindestens ein Fragment eines isolierten natürlichen oder synthetischen Polypeptids enthält, das zur Familie der Cystein-Proteasen vom Kathepsin-L-Typ gehört, das ein apparentes Molekulargewicht von 28 Kilodalton und einen isoelektrischen Punkt aufweist, der im Bereich von 6 bis 9 liegt, wobei das Polypeptid aus der Hornschicht der menschlichen Epidermis isoliert werden kann, **dadurch gekennzeichnet, dass** das Polypeptid und seine Fragmente an der Verringerung der interkorneozytären Kohäsion beteilt sind, indem sie das Corneodesmosin abbauen.

2. Zusammensetzung nach dem vorhergehenden Anspruch, **dadurch gekennzeichnet, dass** das Polypeptid bei einem pH-Wert, der im Bereich von 2 bis 9 und vorzugsweise 3,5 bis 6,5 liegt, seine maximale Aktivität hat.

3. Zusammensetzung, **dadurch gekennzeichnet, dass** sie mindestens ein Polypeptid enthält, das zum Teil aus dem Polypeptid besteht, das wie in den Ansprüchen 1 und 2 beschrieben ist.

4. Zusammensetzung nach einem der vorhergehenden Ansprüche, **dadurch gekennzeichnet, dass** sie mindestens ein Fragment des Polypeptids enthält, das wie in den Ansprüchen 1 und 2 beschrieben ist, das durch Proteolyse oder auf synthetischem Weg erhalten wird.

5. Zusammensetzung nach einem der vorhergehenden Ansprüche, **dadurch gekennzeichnet, dass** das Polypeptid oder das Fragment in einer Menge enthalten ist, die im Bereich von 0,00001 bis 50 Gew.-%, vorzugsweise im Bereich von 0,001 bis 10 Gew.-%, bezogen auf das Gesamtgewicht der Zusammensetzung, liegt.

6. Zusammensetzung nach einem der vorhergehenden Ansprüche, **dadurch gekennzeichnet, dass** sie außerdem mindestens einen Protease-Aktivator enthält.

7. Zusammensetzung nach Anspruch 6, **dadurch gekennzeichnet, dass** der Protease-Aktivator unter Glycerin, Harnstoff und seinen Derivaten, der Transglutaminase, EDTA, den Reduktionsmitteln ausgewählt ist.

8. Zusammensetzung nach Anspruch 7, **dadurch gekennzeichnet, dass** sie mindestens einen Protease-Aktivator enthält, bei dem es sich um ein Reduktionsmittel in aktiver Form oder in Form eines seiner Vorläufer handelt, das unter den Sulfiden, den Thiolen, wie Dithiothreit oder Trithiohexit, Cystein, N-Acetylcystein, den Cystein-reichen Proteinen oder Hydrolysaten von Proteinen, Mercaptoethanol, Thioglycerin, den Thioalkansäuren und den Mercaptocarbonsäuren und ihren Analoga, der Mercaptobernsteinsäure, der Thiomilchsäure, der Thioglykolsäure und ihren Salzen, Coenzym A, reduziertem Glutathion (GSH) und Oxothiazolidincarboxylat ausgewählt ist.

9. Zusammensetzung nach einem der Ansprüche 6 bis 8, **dadurch gekennzeichnet, dass** der Protease-Aktivator in einer Menge enthalten ist, die im Bereich von 0,00001 bis 15 Gew.-% und vorzugsweise 0,001 bis 10 Gew.-%, bezogen auf das Gesamtgewicht der Zusammensetzung, enthalten ist.

10. Verwendung eines isolierten natürlichen oder synthetischen Polypeptids oder mindestens eines Fragments eines isolierten natürlichen oder synthetischen Polypeptids, das zur Familie der Cystein-Proteasen vom Kathepsin-L-Typ gehört, das ein apparentes Molekulargewicht von 28 Kilodalton aufweist, für die Herstellung einer kosmetischen Zusammensetzung nach einem der Ansprüche 1 bis 10, **dadurch gekennzeichnet, dass** die Zusammensetzung dafür vorgesehen ist, auf die Haut aufgetragen zu werden, um die übermäßige interkorneozytäre Kohäsion zu bekämpfen oder um die Abschuppung zu fördern.

11. Pharmazeutische Zusammensetzung nach einem der Ansprüche 1 bis 9, die dafür vorgesehen ist, die Störungen der Abschuppung zu behandeln.

12. Pharmazeutische Zusammensetzung nach Anspruch 11, die für die Behandlung der Hyperkeratose vorgesehen ist.

13. Pharmazeutische Zusammensetzung nach Anspruch 11 oder 12, die für die Behandlung der Xerose, der Ichthyosen, der Psoriasis, der gutartigen oder bösartigen Tumorläsionen oder der reaktiven Keratosen vorgesehen ist.

14. Pharmazeutische Zusammensetzung nach Anspruch 11 oder 12, die für die Behandlung der Leukokeratose des Gebärmutterhalses im Laufe des Uterusprolapses, der buccalen Leukokeratosen oder auch der gutartigen Hornhaut-Tumorläsionen der Malpighi-Schleimhäute vorgesehen ist.

15. Isoliertes natürliches oder synthetisches Polypeptid, das zur Familie der Cystein-Proteasen vom Kathepsin-L-Typ gehört, das ein apparentes Molekulargewicht von 28 Kilodalton aufweist, das wie in einer Zusammensetzung nach einem der vorhergehenden Ansprüche beschrieben ist, **dadurch gekennzeichnet, dass** es aus dem menschlichen Stratum corneum isoliert werden kann und dass es einen isoelektrischen Punkt aufweist, der im Bereich von 6 bis 9 liegt.

16. Verwendung des isolierten Polypeptids oder seiner Fragmente, die wie in den Ansprüchen 1 bis 15 beschrieben sind, zur Herstellung oder Reinigung jedes Moleküls, das imstande ist, spezifisch an das isolierte Polypeptid oder an die isolierten, durch Proteolyse erhaltenen Fragmente oder an das synthetische Peptid zu binden.

17. Verwendung nach dem vorhergehenden Anspruch zur Herstellung oder Reinigung spezifischer Strukturproteine der Korneodesmosomen der Hornhaut.

18. Verwendung des isolierten Polypeptids, das wie in den Ansprüchen 1 bis 4 oder 15 beschrieben ist, für die Herstellung oder Reinigung von Antiseren oder spezifischen monoklonalen Antikörpern.

## Claims

1. Cosmetic or pharmaceutical composition comprising, in a physiologically acceptable medium, at least one polypeptide or at least one fragment of a natural or synthetic isolated polypeptide, belonging to the family of cathepsin L type cysteine proteases, having an apparent molecular weight of 28 kilodaltons and an isoelectrical point of between 6 and 9, it being possible for the said polypeptide to be isolated from the horny layer of the human epidermis, **characterized in that** the said polypeptide and its fragments are involved in the reduction of intercorneocyte cohesion by degrading corneodesmosin.

2. Composition according to the preceding claim, **characterized in that** the polypeptide activity is maximal at a pH of between 2 and 9, preferably between 3.5 and 6.5.

3. Composition, **characterized in that** it comprises at least one polypeptide partly consisting of the polypeptide as described in Claims 1 and 2.

4. Composition according to one of the preceding claims, **characterized in that** it contains at least one fragment of the polypeptide as described in Claims 1 and 2 obtained by proteolysis or synthetically.

5. Composition according to one of the preceding claims, **characterized in that** the polypeptide or fragment is in a quantity of between 0.00001% and 50%, preferably of between 0.001% and 10% by weight relative to the total weight of the composition.

6. Composition according to any one of the preceding claims, **characterized in that** it comprises, in addition, at least one protease activator.

7. Composition according to Claim 6, **characterized in that** the protease activator is chosen from glycerol, urea and its derivatives, transglutaminase, EDTA and reducing agents.

8. Composition according to Claim 7, **characterized in that** it comprises at least one protease activator which is a reducing agent in active form or in the form of their precursor, chosen from sulphides, thiols such as dithiothreitol or trithiohexitol, cysteine, N-acetylcysteine, proteins or protein hydrolysates rich in cysteine, mercaptoethanol, thioglycerol, thioalkanoic acids and mercaptocarboxylic acids and analogues thereof, mercaptosuccinic acid, thiolactic acid, thioglycolic acid and their salts, coenzyme A, reduced glutathione (GSH) or oxothiazolidine carboxylate.

9. Composition according to any one of Claims 6 to 8, **characterized in that** the protease activator is in a quantity of between 0.00001% and 15% and preferably between 0.001% and 10% by weight relative to the total weight of the composition.

10. Use of a polypeptide or of at least one fragment of a natural or synthetic isolated polypeptide, belonging to the cathepsin L type cysteine proteases, having an apparent molecular weight of 28 kilodaltons, for the preparation of a cosmetic composition according to one of Claims 1 to 10, **characterized in that** the composition is intended to be applied to the skin for combating intercorneocyte cohesive excesses or for promoting desquamation.

11. Pharmaceutical composition according to any one of Claims 1 to 9 for treating desquamation disorders.

12. Pharmaceutical composition according to Claim 11 for treating hyperkeratosis.

13. Pharmaceutical composition according to either of Claims 11 and 12 for treating xerosis, ichthyoses, psoriasis, benign or malignant tumour lesions or reactive keratoses.

14. Pharmaceutical composition according to either of Claims 11 and 12 for treating leukokeratosis of the uterine neck during prolapsus, buccal leukokeratoses or keratotic benign tumour lesions of the Malpighian mucosae.

15. Natural or synthetic isolated polypeptide, belonging to the cathepsin L type cysteine proteases, having an apparent molecular weight of 28 kilodaltons, as described in a composition according to one of the preceding claims, **characterized in that** it can be isolated from the human stratum corneum and **in that** it has an isolectric point of between 6 and 9.

16. Use of the isolated polypeptide or of its fragments as described in Claims 1 to 15 to prepare or purify any molecule capable of specifically bonding to the said isolated polypeptide or to the said isolated proteolysis fragments or to the said synthetic peptide.

17. Use according to the preceding claim to prepare or purify structural proteins specific to corneodesmosomes.

18. Use of the isolated polypeptide as described in Claims 1 to 4 or 15, to prepare or purify specific monoclonal antibodies or antisera.
